# EUROPÄISCHE PATENTSCHRIFT

(11) **EP 0 414 808 B1**
(45) Veröffentlichungstag und Bekanntmachung des Hinweises auf die Patenterteilung: **17.04.1996**
(21) Anmeldenummer: 89906669.0
(22) Anmeldetag: 13.06.1989
(51) Int. Cl.: C12N 15/29, G01N 33/68, G01N 33/577, A01N 65/00

(54) **VERFAHREN ZUM SCREENEN EINER EXPRESSIONS-cDNA-KLONBANK ZUR AUFFINDUNG VON POLYNUKLEOTIDEN**
PROCESS FOR SCREENING AN EXPRESSION cDNA CLONE BANK FOR POLYNUCLEOTIDES
PROCEDE D'ANALYSE D'UNE BANQUE DE CLONES D'ADNc D'EXPRESSION POUR TROUVER DES POLYNUCLEOTIDES

(30) Priorität: 14.10.1988 AT 2554/88
(43) Veröffentlichungstag der Anmeldung: 06.03.1991
(73) Patentinhaber: BIOMAY BIOTECHNIK PRODUKTIONS- UND HANDELSGESELLSCHAFT M.B.H., A-4020 Linz (AT)
(72) Erfinder: BREITENBACH, Michael, A-1120 Wien (AT); KRAFT, Dietrich, A-1170 Wien (AT); RUMPOLD, Helmut, A-1180 Wien (AT); SCHEINER Otto, A-2380 Perchtoldsdorf (AT); BREITENEDER, Heimo, A-1190 Wien (AT); PETTENBURGER, Karin, A-1040 Wien (AT); VALENTA, Rudolf, A-2604 Theresienfeld (AT)
(74) Vertreter: Itze, Peter, Dipl.-Ing.
(86) Internationale Anmeldenummer: AT8900058
(87) Internationale Veröffentlichungsnummer: WO9004025

(56) Entgegenhaltungen:
- JOURNAL OF ALLERGY & CLINICAL IMMUNOLOGY, Band 81, Nr. 1, Januar 1988, St. Louis, MO (US); T.W. HATTON et al., Seite 183, Nr. 58
- INT. ARCH. ALLERGY APPL. IMMUNOLOGY, Band 87, Nr. 1, 1988, Karger AG, Basel (CH); H. BREITENEDER et al., Seiten 19-24
- JOURNAL OF ALLERGY & CLINICAL IMMUNOLOGY, Band 72, Nr. 2, August 1983, St. Louis, MO (US); H. IPSEN et al., Seiten 150-159
- THE EMBO JOURNAL, Band 8, Nr. 7, Juli 1989, IRL Press; H. BREITENEDER et al., Seiten 1935-1938
- INT. ARCHS. ALLERGY APPL. IMMUNOLOGY, Band 85, 1988; W.R. THOMAS et al., Seiten 127-129
- PROCEEDINGS OF THE NATL. ACADEMY OF SCIENCES USA, Band 85, 1988, Washington, DC (US); K. SI YUN FANG et al., Seiten 895-899
- JOURNAL OF EXPERIMENTAL MEDICINE, Band 167, 1988; K.Y. CHUA et al., Seiten 175-182

## Beschreibung

Die Erfindung bezieht sich auf ein Verfahren zum Screenen einer Expressions-cDNA-Klonbank zur Auffindung von Polynukleotiden, die in ihrem offenen Leserahmen für Proteine kodieren, deren biologische Aktivität als Allergene den in der Natur vorkommenden Pflanzenallergenen entsprechen.

Mindestens 10% der Bevölkerung leiden zu verschiedenen Zeiten des Lebens und in verschiedenem Ausmaß an Pollenallergien. Die Patienten klagen zur Zeit des Pollenfluges über Juckreiz in der Nase, juckende und gerötete Augen, Schnupfen, Lidödeme, öfters auch Husten und Asthmabeschwerden. Vorwiegend leichte, durch Wind übertragene Pollen cringen zu den Schleimhäuten der Augen und des Respirationstraktes der Menschen vor, werden lokal teilweise aufgelöst und führen bei Patienten mit genetischer Disposition, sogenannten Atopikern, zur Sensibilisierung und damit zu erhöhter Produktion von IgE-Antikörpern gerichtet gegen verschiedene Proteine aus den Pollen. Es handelt sich dabei in den Monaten Februar bis April um die Pollen von Bäumen, wie z. B. Erle, Hasel, Birke, in den Monaten Mai, Juni und Juli, um die Pollen von Gräsern und Getreiden sowie in den Monaten Juli und August um die Pollen von Unkräutern, wie Beifuß, Wegerich, Ampfer und Gänsefuß. Bei neuerlichem Kontakt verursachen die Pollenproteine (Allergene) durch Verbindung mit IgE-Molekülen an der Oberfläche von Mastzellen in Schleimhäuten eine Freisetzung von Entzündungsstoffen, wie Histamin, Leukotrienen, chemotaktischen Faktoren, plättchenaktivierendem Faktor (PAF) u. a., und führen zu einem typischen Krankheitsbild (Heuschnupfen, Pollenasthma), welches als allergische Reaktion vom Typ I bezeichnet wird.

Die unangenehmen bis gefährlichen Wirkungen einer Pollenallergie werden seit Jahrzehnten mit antiinflammatorischen Medikamenten und/oder mit Hilfe der sogenannten Hyposensibiisierung behandelt. Letztere besteht in der Zufuhr von krankheitsauslösenden Pollenproteinen in langsam steigender Dosierung in Form von Injektionen, oder, bei Kindern, in der Gabe von Tropfen bis zur Erreichung einer deutlichen Abnahme der Symptome (Eintreten einer Toleranz). Diese Form der Immuntherapie gilt auf Grund ihrer Erfolge als Basis jeder Therapie einer Pollenallergie mit ausgeprägter Symptomatik. Voraussetzung für einen guten Erfolg für derartige Behandlungen ist allerdings, daß eine Diagnostik, die Hautteste, serologische Teste u. dgl. umfaßt, mit genau definierten Pollenextrakten vorgenommen werden kann, und daß die Behandlung mit dem die Pollenallergie auslösenden Proteinen in adäquaten, exakt zu bestimmenden Konzentrationen vorgenommen wird. Bisher werden die Pollenproteine dadurch erhalten, daß durch aufwendige Verfahren die Pollen gesammelt werden, wobei zu bedenken ist, daß die gesammelten Pollen in der Regel eine Mischung unterschiedlichster Pflanzenpollen darstellen, die anschließend in aufwendigen Verfahren entmischt, gereinigt und aufgearbeitet werden müssen. Weiters ist es mit den bekannten Verfahren nahezu unmöglich, größere Mengen eines reinen Pollen-proteins zu erhalten. Die eine Allergie hervorrufenden Proteine sind nämlich in unterschiedlichsten Konzentrationen an und in den Pollen zugegen, oft abhängig von Klima, Jahreszeit und Wetter. Daher wird bei Verwendung von nicht standardisierten Extrakten in vielen Fällen den individuellen Gegebenheiten der Patienten zu wenig Rechnung getragen, was sich in mangelhaften Therapieerfolgen widerspiegelt.

Breiteneder H. et al., 1988, Int. Arch. Allergy Appl. Immunol Vol 87, S.19-24, hat schon die Isolierung und Charackterisierung vom in RRA aus Betula Verrucosa pollen mittels IgE Bindung von in Vitro Transiliationsprodukten offenbart, jedoch weder ein Verfahren zur Auffindung von pollenallergenen Kodierenden cDNA's offenbart noch suggeriert.

Der Erfindung liegt die Aufgabe zugrunde, ein Verfahren der eingangs genannten Art zu schaffen, mit welchem die für allergische Reaktionen einsetzbaren Allergene bzw. die für sie kodierenden Polynukleotide auf einfache und spezifische Weise aufzufinden sind.

Erfindungsgemäß wird diese Aufgabe dadurch gelöst, daß die in der Expressions-cDNA-Klonbank exprimierten Proteine mittels aus Allergikerseren stammenden IgE-Antikörpern identifiziert werden. Dadurch werden gezielt jene Proteine und die für sie kodierenden Polynukleotide erfaßt, die für die allergischen Reaktionen verantwortlich sind.

Der cDNA-Klon, der für das im folgenden beschriebene Hauptallergen der Birke, *Bet v* I, codiert, ist in hohem Maße homolog zum dem Krankheitsresistenz der Erbse hervorrufenden Gen (55% Identität, 70% Homologie der Sequenz ohne Lücken). Das Erbsengen ist in gesundem Gewebe nicht aktiv, wird jedoch bei Kontakt mit Pflanzenpathogenen in großer Menge exprimiert. Es ist daher zu erwarten, daß dieses in hohem Maße konservierte Gen zum Schutz transgener Pflanzen gegen Planzenpathogene eingesetzt werden kann.

### METHODIK

### I) RNA-Isolation wurde durchgeführt aus:

1) Blättern
2) Infloreszenzen, Wurzeln und Kallusgewebe
3) Pollen
   von *Betula verrucosa*

### 1) ) Blatt RNA-Extraktion

5g Blattmaterial, aufbewahrt bei minus 70° C oder frisch gepflückt und in flüssigem N₂ transportiert, wurde in einer mit N₂ gekühlten Mühle zu Staub zermahlen. Dieser wurde in eine Reibschale transferiert und folgende Lösungen wurden hinzugefügt:
1) 8 ml Tris(hydroxymethyl)-aminomethan/Natrium-docdecylsulfat (Tris/SDS) Puffer (0,1 M Tris.HCl pH 8.0, 1% w/v SDS)
2) 4 ml mit 1 M Tris.HCl, pH 8,0, gepuffertes Phenol
3) 4 ml CHCl₃/Isoamylalkahol (24:1) = CI
   Das zerriebene Gewebe wurde in dieser Hischung (1-3) zu einer feinen Suspension aufgerührt, in Corex-Röhrchen transferiert, kräftig gemischt und in einer Sorvallzentrifuge 5 Minuten bei 6800 g und 4° C zentrifugiert. Es folgte eine nochmalige PCI(Phenol-CHCl₃-Isoamylalkohol)-Extraktion der wäßrigen Phase, eine Phasentrennung durch Zentrifugation bei 3000 g für 5 Minuten und nochmalige CHCl₃-Extraktion der wäßrigen Phase. Zu dieser wurden 10 vol% 2M CH₃COONa (NaAc) pH 5,8 und 250 vol% absolutes Ethanol (EtOH) zugegeben und die gesamten Nukleinsäuren über Nacht bei-20° C gefällt.

Diese wurden 10 Minuten in einem Beckmann JS13-1 "swing out" Rotor bei 3000 g und 4° C abzentrifugiert, der überstand verworfen, das Pellet mit 70% EtOH gewaschen und in einem Exsiccator getrocknet. Das Pellet wurde anschließend in 2 ml H₂O aufgelöst und in Eppendorfgefäße transferiert. Darauf folgt die Zugabe von 150 mg festem NaCl/ml und Aufbewahrung der Lösung bei 4° C für 5 Stunden.

Die nach dieser Zeit ausgefallene RNA wurde bei 4° C und 15.000 g in einer Eppendorf Zentrifuge pelletiert. Das Pellet wurde mit 0,5 ml 2,5 M Nacl gewaschen, die RNA wiederum wie oben pelletiert und der Waschvorgang wiederholt. Anschließend wurde das Pellet dreimal mit 0,5 ml 70% EtOH gewaschen, getrocknet und in 360 ul sterilem Wasser gelöst. Die Fällung der RNA erfolgte durch Zugabe von 40 ul 2M NaAc, pH 5,8 und 1 ml absolutem EtOH bei -20° C über Nacht. Die RNA wurde wiederum pelletiert, das Pellet zweimal mit 0,5 ml 70% EtOH gewaschen, getrocknet und in 100 ul sterilem Wasser gelöst. Die Aufbewahrung der RNA erfolgte bei -20° C.

### 2) Infloreszenzen Wurzelmaterial und Kallusgewebe: Cetyltri-methylammoniumbromid (CTAB)-Methode

30 g Pflanzenmaterial wurde in flüssigem N₂ zerrieben und mit gleichem Volumen kochenden Extraktionspuffer (2% CTAB, 100 mM Tris.HCl, pH 7,8, 20 mM EDTA, 1,4 M NaCl, 1% beta-Mercaptoäthanol) übergossen. Die Temperatur der Lösung wurde im Wasserbad unter Rühren auf 50° C gebracht, das Gemisch in SS-34 Zentrifugenröhrchen transferiert und mit gleichem Volumen CHCl₃:Isoamyl-alkohol (24:1, CI) versetzt und vorsichtig vermengt. Zentrifugation erfolgte 10 Minuten bei 17.400 g in einer Sorvall SS-34 Zentrifuge bei Raumtemperatur. Die wäßrige Phase wurde in ein zweites Röhrchen transferiert und ein Zehntel Volumen 10% CTAB-Lösung zugegeben (10% CTAB, 0,7 M NaCl). Darauf folgte eine nochmalige CI-Extraktion. Zur abgehobenen wäßrigen Phase wurde ein gleiches Volumen Präzipitationspuffer (1% CTAB, 50 mM Tris, 10 mM EDTA, pH 8,0) zugegeben und gut gemischt. Die Lösung wurde 30 Minuten bei Raumtemperatur stehengelassen, dann die Nukleinsäuren in einem SS-34 Rotor für 5 Minuten bei 3000 g abzentrifugiert. Das Pellet wurde in 10 ml 1 M gepufferter CsCl Lösung (50 mM Tris, 5 mM EDTA, 50 mM NaCl, pH 8,0) gelöst. Es erfolgte eine Zentrifugation über einem Polster aus 5,7 M gepuffertem CsCl (2 ml, 50 mM Tris, 5 mM EDTA, 50 mM NaCl, pH 8,0) für 18 bis 20 Stunden bei 120.000 g in einem "swingout" Rotor. Die RNA fand sich im Pellet, wurde in sterilem H₂O gelöst, gefällt und bei-20° C aufbewahrt.

### 3) RNA-Isolation aus Pollen

500 mg Pollen wurden in einer Reibschale mit feinstzerriebenem Glasstaub unter flüssigem N₂ zermahlen. Es erfolgte eine sofortige Zugabe von: 10 ml PCI, 10 ml Homogenisierungspuffer (10 mM Tris, 200 mM NaCl, 5 mM HgCl₂, pH 9,0) und 0,5 ml 20 % SDS. Es wurde bis zur Verflüssigung des anfangs noch gefrorenen Gemisches ständig weitergerieben. Dieses Gemisch wurde dann in ein SS-34 Zentrifugationsröhrchen transferiert und auf Eis gestellt. Die Reibschale wurde mit 5 ml Homogenisierungspuffer und 1 % SDS nachgewaschen. Die Lösung wurde in Zentrifugationsröhrchen unter ständiger Zwischenkühlung auf Eis 10 Minuten kräftig gemischt und danach 10 Minuten bei 3.000 g und 4° C zentrifugiert. Es folgte eine zweimalige PCI- und eine einmalige CI-Extraktion der wäßrigen Phase. Fällung der Nukleinsäuren erfolgte durch Zugabe von 10 vol% 3 M NaAc, pH 4,8 und 250 vol% absolutem EtOH bei-20° C über Nacht. Nach 10 minütiger Zentrifugation bei 12.000 g und 4° C wurde das Pellet einmal mit 70% EtOH gewaschen, ohne es von der Wand des Zentrifugationsröhrchens abzulösen und in geringer Menge H₂O gelöst und auf Eppendorfgefäße verteilt. Eine erneute Fällung erfolgt durch Zugabe von 10 vol% 3 M NaAc, pH 4,8 und 250 vol% absolutem EtOH.

Anreicherung von poly (A)⁺RNA in allen Fällen (1, 2 und 3) erfolgte nach folgender Methode:
Die RNA wurde für 10 Minuten in einer Eppendorf Zentrifuge bei 15.000 g und 4°C aus der Fällung abzentrifugiert. Das Pellet wurde in einem Exsiccator 10 Minuten getrocknet und anschließend in 300 ul sterilem H₂O auf Eis resuspendiert.

Oligo dT-Zellulose wurde in 0,1 M KOH gut suspendiert. Diese Suspension wurde in eine 1 ml fassende, mit Quarzwolle verschlossene Plastiksäule gegossen und diese zu 3/4 gefüllt. Anschließend wurde die Säule mit 5 ml 0,1 M KOH gewaschen und mit Wasser bis zur Erreichung eines neutralen pH-Wertes gespült. Die Säule wurde mit 4 ml Ladepuffer (0,5 M LiCl, 10 mM Tris.HCl, pH 7,5, 1 mM EDTA, 0,1% SDS) äquilibriert.

Die RNA-Probe wurde mit einer 5 M LiCl-Lösung auf eine 0,5 M LiCl-Konzentration eingestellt, bei 60°C für 10 Minuten denaturiert und dann auf Trockeneis rasch abgekühlt. Die Probe wurde auf die Säule aufgetragen, mit 1 ml Ladepuffer nachgewaschen und nochmals auf die Säule aufgetragen. Anschließend wurde die Säule mit 5 ml middle rinse" Puffer (10 mM Tris.HCl, pH 7,5, 1 mM EDTA, 0,15 mM LiCl, 0,1% SDS) gespült.

Die Elution der poly(A)⁺ RNA-haltige Fraktion erfolgte durch Spülen der Säule mit 8 x 300 ul Portionen von auf 60°C erwärmtem Elutionspuffer (2 mM EDTA, pH 8,0, 0,1% SDS). Die RNA wurde durch Zugabe von 3 M NaAc, pH 4,8, zu einer Konzentration von 0,3 M und 300 vol% absolutem EtOH bei -20°C über Nacht gefällt.

### II) RNA-Charakterisierung

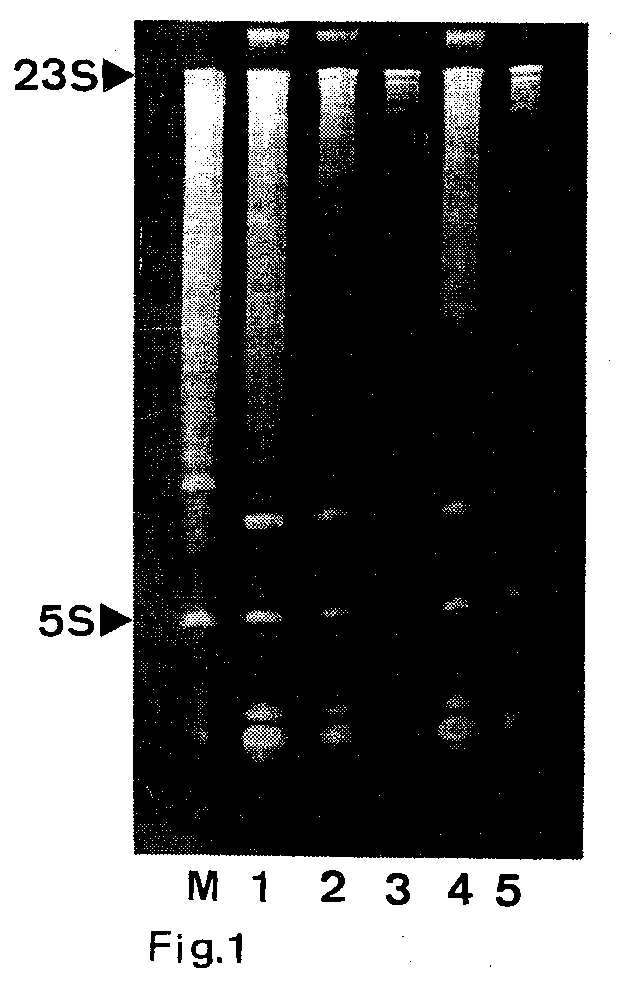

erfolgte durch
a) Auftragen der Gesamt-RNA auf ein Polyacrylamid(6%)-Harnstoff(50%)gel und Elektrophorese unter Verwendung von 5S-, 16S-und 23S-RNAs als Vergleichsubstanzen (Fig. 1).
b) Bestimmung der poly(A)⁺ RNA-haltigen Fraktion nach oligo-dT-Säule erfolgte durch Auftragen von Aliquots auf 1% Agaroseplatten mit 0,5 ug Ethidiumbromid/ml und Sichtbarmachung der Dots auf einem UV-Transilluminator.

### III) cDNA-Synthese

1 ug poly(A)⁺ RNA wurde zur cDNA-Synthese in folgender Reaktion eingesetzt:
a) Synthese des ersten Stranges:
   4 ul 5 x Puffer für die Synthese des ersten Stranges (250 mM Tris.HCl, pH 8,3, 250 mM KCl, 50 mM MgCl₂, 50 mM Dithiothreit)
   1 ul 20 mM Natriumpyrophosphat
   1 ul humaner plazentaler RNAse-Inhibitor (20 U/ul)
   2 ul Desoxynukleotidtriphosphat-Mix (dATP, dGTP, dTTP: 10 mM; dCTP: 5mM)
   1 ul oligo dT (12-18) 1,6 mg/ml
   0,5 ul alpha ³P-dCTP (5 uCi)
   H₂O ad 20 ul

   Nach Mischen erfolgte die Zugabe von 20 U reverser Transkriptase und danach Inkubation bei 42° C für 60 Minuten. Die Messung der Synthese des ersten Stranges ergab einen typischen Einbau von 100.000 cpm/ug RNA.
b) Synthese des zweiten Stranges
   20 ul Reaktionsgemisch von a)
   20 ul 5x Puffer für die Synthese des 2. Stranges (100 mM Tris.HCl , pH 7,5, 0,5 M KCl, 25 mM HgCl₂, 50 mM (NH₄)₂SO₄, 50 mM Dithiothreit)
   5 ul alpha ³P-dCTP (50 uCi)
   0,8 U Ribonuclease H aus *E. coli*
   23 U DNA Polymerase I aus *E. coli*
   H₂O ad 100 ul

Mischen und Inkubieren: 12° C für 60 Minuten, 22° C für 60 Minuten, danach 70° C für 10 Minuten. Es folgte ein Abkühlen des Reaktionsgemisches auf Eis, Zugabe von 2,0 U T4 DNA-Polymerase und Inkubation bei 37° C für 10 Minuten (Hier konnte wie oben ein Aliquot zur Messung des radioaktiven Einbaues in den 2. Strang entnommen werden; es ergab sich ein Einbau von 90% des Einbaues in den ersten Strang).

Im weiteren erfolgte eine 2 malige PCI-Extraktion und eine einmalige CI-Extraktion der doppelsträngigen cDNA. Gefällt wurde mit dem gleichen Volumen 4 M NH₄-Acetat und 200 vol% absolutem EtOH für 20 Minuten bei -70° C. Die cDNA wurde für 10 Minuten bei 15.000 g und 4° C abzentrifugiert, das Pellet in 100 ul sterilem H₂O gelöst und nochmals - wie oben angegeben - gefällt. Das wiederum abzentrifugierte Pellet wurde mit 500 ul 70% EtOH gewaschen, 5 Minuten wie oben zentrifugiert und in 20 ul ster. H₂O gelöst.

### IV) CDNA Klonierung in lambda gt 11

### 1) Methylierung der cDNA zum Schutz interner Eco RI-Restriktionsstellen. Reaktionsgemisch:

1 ug cDNA in 10 ul sterilem H₂O
4 ul 5 x Eco RI-Methylase-Puffer (250 mM Tris.HCl, pH 7,5, 5 mM EDTA, 25 mM Dithiothreit)
2 ul 100 uM S-adenosyl-L-methionin (Stocklösung: 10 mM S-adenosyl-L-methionin in 10 mM CH₃COONa-Puffer, pH 5,0. Verdünnung 1:10⁻ in sterilem H₂O kurz vor Gebrauch).
4 ul steriles H₂O
20 U Eco RI Methylase
Inkubation bei 37° C für 60 Minuten, Enzymaktivierung bei 70° C für 10 Minuten.

### 2) Ligation der Eco RI-Linker: 5'd(pGGAATTCC), 500 ug/ml. Linkerligation:

20 ul Methylierungsreaktion von 1)
3 ul 10x Ligationspuffer (500 mM Tris.HCl, pH 7,5, 100 mM MgCl₂, 200 mM Dithiothreit, 50 mM ATP, 50 ug /ml bovines Serumalbumin)
2 ul Eco RI-Linker (0,5 ug/ul)
5 ul steriles H₂O
5 U T4 DNA-Ligase
Inkubation bei 15° C 16-20 Stunden. Mit Hilfe der T4 DNA-Ligase wurden die Eco RI-Linker an beiden Enden der cDNA ligiert.

### 3) Verdau der Eco RI gelinkerten cDNA mit Eco RI.

Dadurch wurde ein einziges Eco RI "sticky end" an jedem Terminus der cDNA geschaffen und überschüssige Linkermoleküle entfernt. Reaktion:
30 ul Reaktionsgemisch aus 2)
10 ul 10x Eco RI Puffer
60 ul steriles H₂O
100 U Eco RI (1M Tris.HCl, pH 7,5, 0,5 M NaCl, 0,1 M MgCl₂)
Inkubation bei 37° C für 5 Stunden, anschließende Enzyminaktivierung bei 70° C für 10 Minuten.

### 4) Abtrennen der cDNA von überschüssigen Linkermolekülen

Vor der Insertion der cDNA in lambda gt 11 müssen die überschüssigen Linkermoleküle abgetrennt werden, um nicht mit der Klonierung zu interferieren. Zur Trennung wurden kommerziell erhältliche Säulen verwendet, die mit 6 ml STE Puffer (5,84 g NaCl, 1,21 g Tris, 0,37 g EDTA/1, pH 8,0) gewaschen und äquilibriert wurden. 100 ul mit Eco RI verdaute und gelinkerte cDNA wurden auf die Säule aufgetragen. Es wurden 200 ul Portionen mit STE Puffer von der Säule eluiert und separat in Eppendorfgefäßen aufgefangen. Die Aktivität der einzelnen Proben wurde gezählt (Cerenkov) und die Fraktionen mit dem höchsten Zählergebnis vereinigt. Die Fällung der cDNA dieser Fraktionen erfolgte durch Zugabe von 10 vol% 3 M NaAc und 250 vol% absolutem EtOH bei -20° C über Nacht. Die gefällte cDNA wurde 30 Minuten in einer Eppendorferzentrifuge bei 15.000 g abzentrifugiert und in sterilem H₂O zu einer Konzentration von 50 ng/ul gelöst.

### 5) Insertion der mit Eco RI-Enden versehenen cDNA in lambda gt 11

Die lambda gt 11 DNA war bereits mit Eco RI geschnitten und mit alkalischer Phosphatase dephosphoryliert (Clontech RI-lambda gtll, Cat. No. 6331-1) und somit für die Ligationsreaktion bereit:
200 ng cDNA
   1 ug lambda gt 11 Arme
   1 ug 10x Ligationspuffer (wie unter IV, 2.)
H₂O ad 10 ul
2.5 U T4 DNA Ligase
Die Inkubation erfolgte bei 14° C über Nacht.

### 6) Invitro - packaging des Ligationsgemisches erfolgte mit Gigapack Plus (Stratagene, Catalogue No. 200211).

Rasches Auftauen der beiden Extrakte (Sonic extract = SE, vom induzierten "prehead donor" BHB 2690; Freeze / Thaw extract = FTE, vom induzierten "packaging protein donor" BHB 2688). Der gesamte Ligationsansatz wurde zum aufgetauten FTE gegeben und 15 ul SE dazu pipettiert und mit der Pipette vorsichtig gemischt. Inkubation für 2 Stunden bei Raumtemperatur. Danach erfolgte die Zugabe von 500 ul Phagenverdünnungspuffer (500 mg NaCl, 200 mg MgSO₄, 5 ml 1 M Tris. HCl, pH 7,5). Aufbewahrung der Phagensuspension erfolgte bei 4° C.

### 7) Vorbereitung der E. coli Y 1090 Wirtszellen

*E. coli Y* 1090 (ATCC no. 37196 = *E. coli* delta lac.U.169proA⁺ delta lon araD139 strA hflA150 (chr::Tn10) (pMC9)) wurde auf einer LB-amp Platte (1000 ml bestehend aus 10 g Trypton, 5 g Hefeextrakt, 10 g NaCl, 15 g Agar-Agar, 100 mg Ampicillin, pH 7,5) ausgestrichen und über Nacht bei 37° C inkubiert. Einzelkolonien wurden gepickt und in 4 ml LB-amp-Medium unter Zusatz von 0,4 % Maltose über Nacht bei 37° C inkubiert. Die über-Nacht-Kultur wurde abzentrifugiert und in 1 ml 10 mM HgSO₄ resuspendiert. Diese Zellen waren für die Phagenadsorption bereit und konnten bei 4° C aufbewahrt werden.

### 8) Titration der Lambda gt 11 Rekombinanten

Es wurde eine Verdünnungsreihe der Phagensuspension (nach dem Packaging) in 10er Schritten angefertigt. Je 100 ul Y 1090 Zellen in 10 mM MgSO₄, wurden mit je 10 ul Phagensuspension der entsprechenden Verdünnung vermischt. Die Präadsorption der Phagen-partikel an die Wirtszellen erfolgte für 20 Minuten bei Raumtemperatur. Anschließend erfolgte die Zugabe von:
a) 2 ul Ampicillin (25 mg/ml)
b) 10 ul 100 mM IPTG (Isopropyl-beta-D-thiogalaktopyranosid, gelöst in sterilem H₂O)
c) 10 ul einer 2%igen X-gal-Lösung (20 mg 5-Bromo-4-chloro-3-indolyl-beta-D-galaktopyranosid in 1 ml Dimethylformamid) pro ml
d) 4 ml 0,6 % Agarose in H₂O (bei 47° C im Wasserbad gehalten)
Rasches Durchmischen und Gießen der Topagarose erfolgte auf vorgewärmte (37° C) LB-amp-Platten. Die Platten wurden 10 Minuten bei Raumtemperatur zur Verfestigung der Topagarose stehen gelassen und anschließend bei 43° C über Nacht inkubiert. Am darauffolgenden Tag erfolgt die Auszählung der weißen Plaques und damit der rekombinanten lambda gt 11 Phagen.

### V) Expression des rekombinanten Proteins und Immunoscreening

Der lambda gt 11 Vektor erlaubt die kontrollierte Expression des rekombinanten Proteins in Form eines Fusionsproteins mit der beta-Galaktosidase.

### 1) Ausplattieren der cDNA-Bank

Y 1090 Wirtszellen wurden wie oben beschrieben vorbereitet, mit Phagen infiziert und auf LB-amp-Platten (Durchmesser 145 mm) so ausplattiert, daß eine Dichte von 20.000 Plaques/Platte erreicht wurde. Die Platten wurden für 3-4 Stunden bei 43° C inkubiert, bis die Plaques in Erscheinung traten.

### 2) Induktion der Proteinexpression

Die Platten mit den Plaques wurden mit Nitrozellulosefiltern (Durchmesser 132 mm, in 10 mM IPTG getränkt und anschließend getrocknet) überlegt und bei 37° C für 3 Stunden inkubiert (Induktion der Expression). Anschließend wurden die Nitrozeilulosemembranen, an denen auch die Fusionsprotein adsorbiert waren, vorsichtig abgezogen.

### 3) Immunoscreening

Screening mit Patienten IgE
i) Abwaschen der Agarosenpartikel: die Nitrozellulosefilter wurden mit 50 ml GP (50 mM Natriumphosphatpuffer, pH 7,5, 0,5% Tween 20, 0,5% w/v Rinderserumalbumin, 0,05% NaN₃) überschichtet und für 5 Minuten bei 200 rpm auf einem Rundschüttler geschwenkt. Der Puffer wurde abgeschüttet und der Vorgang wiederholt.
ii) Absättigung der freien Bindungsstellen erfolgte mit 25 ml GP für mindestens 30 Minuten bei Raumtemperatur unter leichtem Schwenken
iii) 1. Antikörper
   Das ausgewählte Serum eines Allergikers, dessen IgE-Antikörper das Hauptallergen der Birke *(Bet v* I), ein 17,5 kD Protein, erkennen, wurde 1:10 in GP verdünnt; mit dieser Serumverdünnung wurden die Blots über Nacht bei 4° C unter leichtem Schwenken inkubiert.
iv) Waschen der Blots
   3 maliges vorsichtiges Waschen der Blots erfolgte mit je 30 ml GP bei Raumtemperatur, wobei beim letzten Waschvorgang die Blots für mindestens 30 Minuten mit dem Puffer geschwenkt wurden. Anschließend wurde der Puffer abgegossen.
v) Radioaktiver 2. Antikörper
   26 ml Lösung pro Rundfilter bestehend aus:
   2,6 ml ¹⁵J markiertes anti-human IgE (Pharmacia Int., 300 000 cpm/ml)
   23,4 ml GP (s.o.)
   234 ul Gelatine (100 ul/10 ml GP)
   Die Inkubation erfolgte über Nacht bei Raumtemperatur unter leichtem Schwenken.
vi) Waschen der Blots
   Die Blots wurden 3x mit je 30 ml GP gewaschen, wobei beim letzten Waschvorgang die Pufferlösung wieder für 30 Minuten über den Blotsn geschwenkt wurde.
vii) Trocknen der Blots und Exposition
   Nach Trocknung der Blots erfolgte der autoradiographische Nachweis der lambda gt 11 Klone mit dem für *Bet v* I kodieren-dem Insert durch Exposition mit einem Kodak XR Röntgenfilm für 72 Stunden bei -70° C.

### VI) Reklonierung und Analyse der rekombinanten lambda gt 11 Phagen auf DNA-Niveau

### 1) Reklonierung

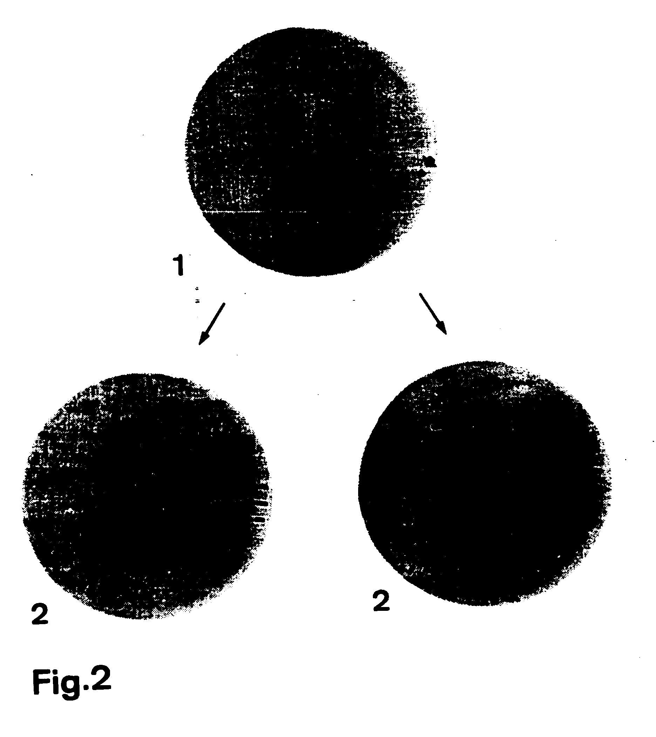

Aufgrund der hohen Plaquedichten mußten 2 Reklonierungsschritte zur Anreicherung der positiven Klone durchgeführt werden. Es wurden Phagenstanzen der positiven Plaques und davon Phagensuspensionen angefertigt. Diese wurden wiederum auf die Konzentration der Phagen titriert. Mit Hilfe des Immunoscreenings wurden erneut die positiven Klone bestimmt. Es konnte eine Anreicherung bis zu 95 % erzielt werden (Fig. 2).

### 2) DNA-Analyse

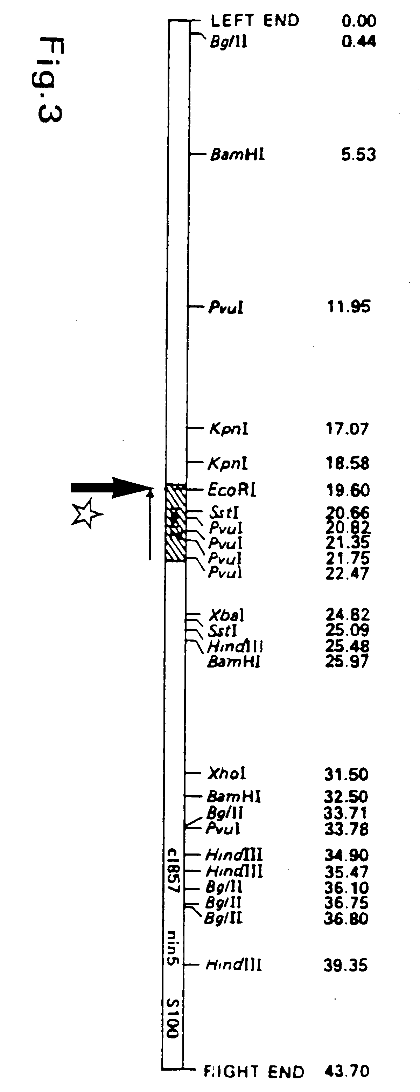

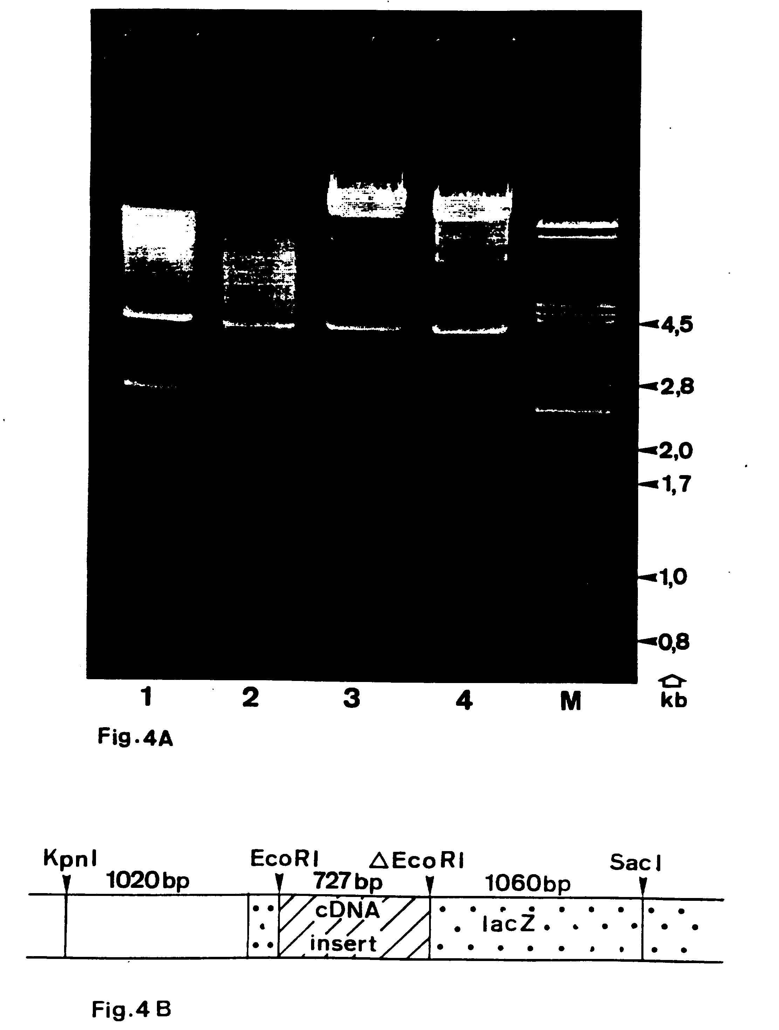

a) Herstellung eines "liquid lysate"
   Ein singulärer Plaque wurde in 500 ul 10 mM MgSO₄ für mindestens 2 Stunden bei 4° C ausgelaugt. 100 ul dieses Phageneluates wurden mit 100 ul *E. coli Y* 1090 Zellen in 10 mM HgSO₄ vermischt und 20 Minuten bei Raumtemperatur zur Adsorption der Phagen an die Wirtszellen stehengelassen. Diese Mischung wurde in 50 ml LB-Medium transferiert. Die Bakterienzellen wurden bei 32° C bis zu einer OD 600 von 0,5-0,6 anwachsen gelassen. Anschließend wurde die Temperatur der Zellkultur im Wasserbad rasch auf 42° C gebracht und für weitere 20 Minuten im Luftbadschüttler bei 42° C gehalten. Danach wurde die Kultur so lange bei 37° C inkubiert, bis die Lyse der Bakterienzellen abgeschlossen war. Eventuell noch intakte Bakterienzellen wurden durch die Zugabe von 10 ul CHCl₃ lysiert. Das Lysat wurde 10 Minuten bei 10.000 rpm in einem SS-34 Rotor abzentrifugiert. Der überstand, der die Phagenpartikel enthielt, konnte bei 4° C aufbewahrt werden. Zu den 50 ml Phagenlysat wurden 10 ul DNAse (5mg/ml) und 25 ul RNAse (10 mg/ml) zugegeben und eine Stunde bei 37° C inkubiert. Die Phagen wurden danach 1,5 Stunden bei 30.000 rpm in einem Beckmann SW-27 Rotor pelletiert. Das Pellet wurde in 200 ul 50 mM Tris.HCl, pH 8,0 resuspendiert. Die Phenolextraktion erfolgte durch Zugabe gleichen Volumens gepufferten Phenols. Die Suspension mußte für 20 Minuten kräftig geschüttelt und anschließend für 2 Minuten abzentrifugiert werden. Die Phenolextraktion war zu wiederholen. Zur wäßrigen Phase wurden 200 ul CHCl₃ zugegeben, gut gemischt und kurz zentrifugiert. Dieser Vorgang war ebenfalls zu wiederholen. Durch die Zugabe von 20 ul 3 M NaAc und 600 vol% absolutem EtOH zur wäßrigen Phase wurde die Phagen-DNA bei Raumtemperatur präzipitiert. Nach 10 minütiger Zentrifugation wurde das Pellet mit 1 ml 70% EtOH gewaschen, dann getrocknet und in 200 ul sterilem H₂O resuspendiert.
b) Restriktionsanalyse
   1 ug lambda gt 11 DNA wurde mit Eco RI verdaut. Da das Insert nicht aus dem Vektor geschnitten werden konnte, mußten die beiden der Insertionsstelle nächstliegenden Restriktionserkennungsstellen gewählt werden: KpnI und SacI (siehe Lambda gt 11 Restriktionskarte, Fig. 3). Dadurch gelang es, ein 2,8 kb großes DNA-Fragment aus dem Vektor zu schneiden (Fig. 4A, Bahn 1; Fig. 4B), das zusätzlich zum Insert rechts und links je 1.000 bp an ursprünglicher lambda-Sequenz enthielt (Fig. 4B). Ein Doppelverdau mit KpnI/Eco RI bzw. SacI/Eco RI zeigte, daß tatsächlich nur eine der beiden Eco RI Erkennungssequenzen verändert worden war, und zwar die der SacI-Schnittstelle näher liegende. Es wurde auf diese Weise ein 1,75 kbp großes Eco RI - SacI Fragment erhalten (Fig. 4A, Bahn 3, mit Doppelkaro markiert). Zum Subklonieren in das Bluescript Plasmid wurde das 2,8 kb KpnI-SacI Fragment verwendet (Fig. 4A, Bahn 1, mit Karo markiert).

### VII) Fusionsprotein und Westernblot

Zum Nachweis der biologischen Aktivität (IgE-Bindungskapazität) des in lambda gt 11 hergestellten Fusionproteins dienten folgende Methoden:

### 1) Herstellun der lysogenenE. coliY 1089

Einzel kolonien von Y 1089 (ATCC no. 37196 = *E. coli* delta lac U169 proA⁺ delta lon araD139 strA hflA 150 (chr::Tn10)(pMC9)) wurden in 10 ml LB amp Medium mit O,4% Maltose inokuliert und über Nacht bei 37° C anwachsen gelassen. 1 ml dieser übernachtkultur wurde in 50 ml auf 37° C vorgewärmtem LB amp - Maltose-Medium transferiert und bis zu einer OD 600 von 0,5 hochgezogen. Danach erfolgte eine Zugabe von 1 vol% 1 M MgSO₄ zur Kultur und eine Aufteilung in 100 ul Portionen. Dazu wurden 50 ul einer Phagenverdünnung (1,25 x 10⁸ plaque forming units/ml) pipettiert. Die Adsorption der Phagen an die Y 1089 Zellen fand für 20 Minuten bei Raumtemperatur statt. Die so infizierten *E. coli* Zellen wurden auf LB amp Platten in einer Dichte von ungefähr 5 Zellen/cm ausplattiert. Die Platten wurden über Nacht bei 32° C inkubiert. Einzelkolonien wurden gepickt und auf 2 separate LB amp Platten ausgestrichen. Eine Platte wurde bei 32° C und eine bei 43° C inkubiert. Lysogene Y 1089 Zellen wuchsen bei 32° C, aber nicht bei 43° C.

### 2) Herstellung eines Proteinextraktes aus den lysosenen Zellen

100 ml LB Medium wurden mit einer Einzelkolonie eines rekombinanten lysogenen *E. coli* Y 1089 inokuliert. Man ließ die Kultur auf eine OD 600 von 0,5 heranwachsen, erhöhte dann die Temperatur rasch auf 42° C. Die Kultur wurde 20 Minuten bei 42° C gehalten. Die Zugabe von IPTG zu einer Konzentration von 10 mM führte zur Expression des Fusionsproteins. Die Kultur wurde 60 Minuten bei 537°C inkubiert, die Zellen anschließend bei Raumtemperatur geerntet. Das Zellpellet wurde in 1/30 des ursprünglichen Kulturvolumens in Phosphatpuffer (50 mM Natriumphosphatbuffer, pH 7,5) resuspendiert und sofort in flüssigem N₂ eingefroren. Auftauen in einem 37° C Wasserbad führte zu einer kompletten Lyse der induzierten lysogenen Bakterienzellen.

### 3) Polyacrylamidgelelektrophorese PAGE und Immunoblot

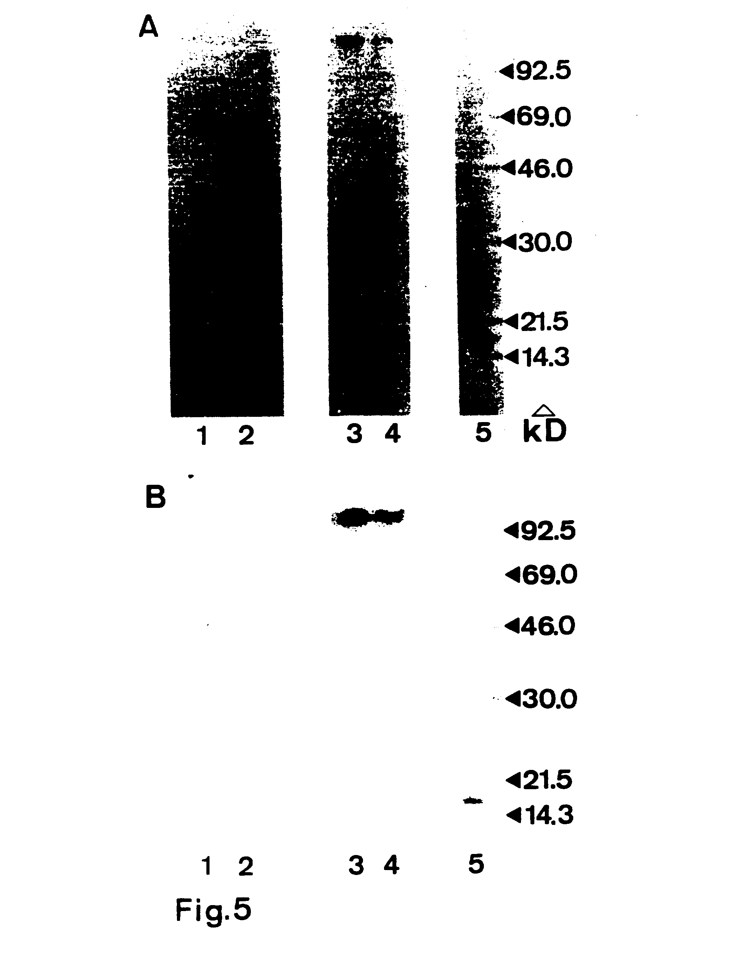

Die biologische Aktivität (IgE-Bindung) des Fusionsproteins wurde mittels Westernblot/Immunoblot erfaßt (Fig. 5A, B). Mittels SDS-PAGE (12%iges homogenes Polyacrylamidgel, 5%iges Stackinggel) aufgetrennte Proteine (500 ug Gesamtproteinextrakt aus rekombinanten lysogenen *E*. *coli* Zellen pro Bahn) wurden im elektrischen Feld bei 150 mA über 4 Stunden auf eine Nitrozellulosemembran transferiert (Transferpuffer: 25 mM Tris.HCl, 192 mM Glycin, 20 % Methanol, pH 8,3). Die Nitrozellulose wurde in Streifen geschnitten, und freie Bindungsstellen wurden 30 Minuten bei Raumtemperatur mit folgendem Puffer abgesättigt: 50 mM Natriumphosphat, pH 7,5, 0,5% Tween 20, 0,5% BSA, 0,05% NaN₃.

Der Nitrozelluloseträger wurde mit Allergikerserum überschichtet, welches in dem Puffer verdünnt war, der dem zur Absättigung der freien Bindungsstellen verwendeten Puffer entsprach. Zur Bestimmung von allergenspezifischem IgE wurde das Allergikerserum in diesem Puffer 1:4 verdünnt. Die Nitrozellulosestreifen wurden jeweils mit dem verdünnten Serum bzw. unverdünnten überstand über Nacht bei 4° C in schaukelnder Bewegung inkubiert. Die so inkubierten Streifen wurden nun dreimal in den für die Absättigung der freien Bindungsstellen verwendeten Puffer gewaschen. Für die Bestimmung des allergenspezifischen IgE wurden die Streifen für 12 Stunden bei Raumtemperatur mit ¹⁵J anti-Human-IgE inkubiert. Dieses anti-IgE wurde im oben angegebenen Puffer 1:4 verdünnt, der zusätzlich 20 mM an Natriumazid war und 0,4 % Gelatine enthielt.

### VIIISubklonierung des 2.8 kb Fragments SacI - KpnI

In der weiteren Folge wurden für die elektrophoretische Isolierung des Insertionsfragmentes, die den beiden Eco-RI-Schnittstellen zunächstgelegenen Schnittstellen, nämlich SacI und KpnI gewählt. Die Abstände zwischen Insert-(Eco RI-) Ende und den beiden Restriktionsendonukleasen SacI und KpnI betragen auf der KpnI-Seite 1020, auf der SacI-Seite 1060 Basenpaare (siehe Fig. 4).

### 1) Isolierung eines DNA-Fragmentes aus einem Agarosegel

a) In zwei Durchgängen wurden auf 1%igen Agarosegelen folgende Verdaue aufgetrennt:
   Je 20 ug recombinante lambda gtll-DNA (plus 2,8 kb Insert) in 20 ul H₂O
   20 ul 10 x Puffer ("low salt P.": 10 mM Tris, pH 7,5, 10 mM MgCl₂, 100 ug/ml BSA)
   60 U KpnI
   160 ul H₂O
   Gesamtvolumen: 200 ul

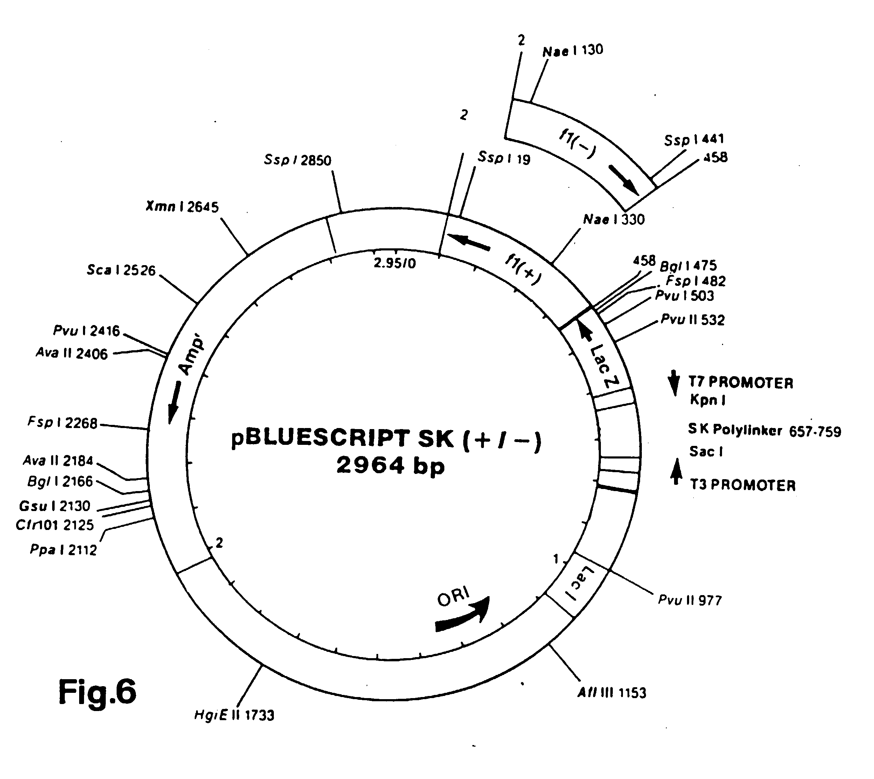

   Die Mischung wurde 3 Stunden bei 37°C inkubiert. Für den unmittelbar anschließenden SacI-Verdau wurde die NaCl-Konzentration durch Zugabe von 2 ul 4 M NaCl in H₂O auf 40 mM eingestellt; dies entspricht annähernd dem für SacI optimalen "Medium Salt Puffer" Mileu. Hierauf wurden 60 U SacI zugegeben und der Verdau weitere 3 Stunden bei 37°C inkubiert.
   Die Größe von 750 bp für das cDNA-Insert ergab sich aus der Differenz der Länge des tatsächlichen Fragmentes von 2830 Basenpaaren mit der Länge des aus der lambda gtll Restriktions-Karte zu berechnenden KpnI-SacI-Abschnittes von 2080 Basenpaaren. Das erhaltene Fragment wurde nun für die genauere Charakterisierung der klonierten cDNA in ein geeignetes "Multipurpose-Plasmid", nämlich das Bluescript-Plasmid (Fig. 6) zwecks Sequenzierung von zirkulärer Doppelstrang- und Einzelstrang-DNA ligiert.
b) Elution des Fragments
   Die im UV-Licht aufgrund der Ethidiumbromideinlagerung gut sichtbaren DNA-Banden auf der Höhe von 2,8 kb wurden auf DE81 Whatman-Filter, die durch Schlitze den Gelfragmenten auf der Anodenseite vorgeschalten wurden, aufgebracht. Die Filterstücke wurden mit je 2x 300 ul low salt Waschpuffer (0,2 M NaCl, 10 mM Tris, 1 mM EDTA, pH 8,0) gewaschen und dann mit 2 x 300 ul high salt Elutionspuffer (1 M NaCl, sonst wie Waschpuffer) daraus die DNA eluiert. Nach 2maliger Phenol- und Etherextraktion der wäßrigen Phase mit anschließender Ethanolfällung über Nacht bei -20°C wurde die DNA pelletiert (15 000 g, 20 Minuten), mit 70%igem Ethanol gewaschen, wieder pelletiert (15 000 g, 10 Minuten) und im Vakuum getrocknet. Die gesamte Menge der eluierten 2,8 kb Fragmente beider Gele wurde in insgesamt 10 ul Wasser gelöst und vereinigt. Ein Aliquot (0,7 ul) wurde zur quantitativen Beurteilung auf ein 1%iges Agarosegel aufgetragen. Die erhaltene Bande von 2,8 kb ließ auf eine Gesamtmenge von 200-300 ng für beide Fragmente bei einer Konzentration von 20-30 ug/ml schließen. Daher wurde die Ligation in Bluescript als Voraussetzung für alle weiteren Charakterisierungsschritte durchgeführt.

### 2) Vorbereitung des Plasmids für die Ligation

### a) Die Bluescript-Plasmid-Subvarianten M13 SK⁺ und M13 SK⁻ (Fig. 6) wurden mit KpnI und SacI verdaut.

Je 2 ug/2 ul Plasmid (SK⁺ bzw. SK⁻) wurden mit je 30 U KpnI und SacI (wie unter VIII 1 a beschrieben) verdaut.

### b) Ligation in M13 SK⁺ und M13 SK⁻

Die verdaute Plasmid DNA wurde (wie unter VIII 1 b beschrieben) gewaschen, pelletiert, getrocknet und in je 5 ul H₂O gelöst. Zur Ligation in das Bluescript-Plasmid wurden zu je 4,8 ul eluiertem und gereinigtem 2,8 kb-Fragment
5 ul Plasmid (SK⁺ bzw. SK⁻)
2 ul dATP (100 mM)
2 ul 10x Ligasepuffer (500 mM Tris, pH 7,4, 100 mM HgCl₂, 10 mM Spermidin)
0,5 ul T4 DNA Ligase (5 U/ul)
6 ul H₂O
pipettiert und der Ligationsansatz 3 Stunden bei Raumtemperatur inkubiert.

### c) Herstellung kompetenter Zellen und Transformation

Für die Transformation wurde ein für das Bluescript-Plasmid geeigneter *E. coli*-Stamm, XLI-Blue (recAI, endAI, gyrA96, thi, hsdR17 (rk⁻mk⁺) supE44, relAI, lambda-, lac-, (F'proAB, lac IqZ delM15, TN10)), gewählt, der eine Selektion Insert-tragender Bluescript-Plasmide durch das beta-Galaktosidase-blau-weiß-Farbindikatorsystem ermöglicht (siehe IV 8).

*E. coli* XLI Blue wurde mit 100 mM CaCl₂ bei 0°C "kompetent" gemacht: 50 ml einer exponentiellen Kultur (XLI Blue in LB-tet (Tetracyclin: 20 mg/l)) wurden bei einer OD von 460 bei 600 nm pelletiert. Das *E. coli*-Pellet wurde zuerst in 50 ml eiskaltem 100 mM CaCl₂ suspendiert, nach einer weiteren Inkubation (20 Minuten bei 0°) abzentrifugiert und in 5 ml 100 mM CaCl₂ resuspendiert. Nach 4 Stunden bei 0°C wurde die Transformation durchgeführt:

Je 1/5 der beiden unter VIII 2 b beschriebenen Ligaseansätze wurde mit 100 ul kompetenter XLI-Blue-Zellen, die anderen 4/5 mit 200 ul kompetenter Zellen bei 0°C eine Stunde inkubiert. Durch einen anschließenden zweiminütigen Hitzeschock der *E. coli*-Bakterien bei 42°C kommt es zu einer Aufnahme der Plasmid-DNA durch die Bakterienwand.
Nach Ausplattieren der Transformationsgemische auf LB-tet-Platten und 18-24 stündiger Inkubation bei 37°C wurden eine Serie weißer Kolonien aus SK⁺ und SK⁻ Transformanten in Vorkultur gebracht. In einer Plasmid-"Mini "-Präparation nach Methode der alkalischen Lyse wurde aus diesen Vorkulturen die Plasmid-DNA isoliert und anschließend durch KpnI/SacI Kontrollverdau das Vorhandensein des 2,8 kb Inserts überprüft. Zwei positive Klone - je eine SK⁺ und eine SK⁻ Variante (2,8 SK⁺ bzw. 2,8 SK⁻) - wurden ausgewählt, um davon eine Plasmid-"Maxi"-Präparation herzustellen.

### d) Plasmid-"Maxi"-Präparation von 2,8 SK⁺ und 2,8 SK⁻

Die Plasmidpräparation nach Methode der alkalischen Lyse wurde aus 300 ml XLI-Blue-Transformanten beimpftem LB-Medium hergestellt. Eine Trennung der Plasmid-DNA von der RNA wurde durch differentielle Polyethylenglycol (PEG) Fällung mit anschließender Phenolätherextraktion und Isopropanolfällung vorgenommen.

### IX. Sequenzierung des 2,8 kb Fragmentes mit Sequenase

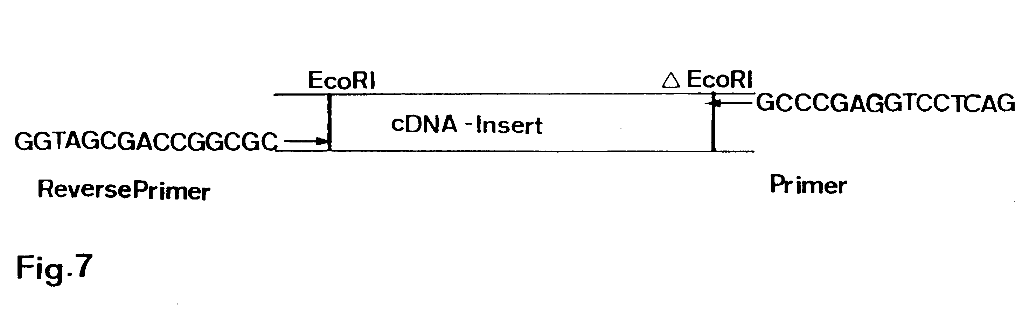

Für die Sequenzierung wurden 2 käuflich erworbene lambda gt11-Primer als Ausgangspunkt der enzymatischen Synthese von Komplementärstrangabschnitten verwendet.
1.) Ein 15 Basen langes Einzelstrangnukleptid mit der Sequenz 5'.... G A C T C C T G G A G C C C G .... 3', vom Hersteller als lambda gt11-Primer bezeichnet, das 12 Basen von der EcoRI-Insertionsstelle entfernt auf dem der SacI Schnittstelle zugekehrten lambda gt11-Arm anlagert (Fig. 7)
2.) Ein 15 Basen langes Einzelstrangnukleotid mit der Sequenz 5'.... G G T A G C C A C C G G C G C .... 3', als lambda gt11 Reverse-Primer bezeichnet, das 7 Basen von der Eco RI-Schnittstelle entfernt auf dem der KpnI Schnittstelle zugekehrten lambda gt11-Arm anlagert (Fig. 7).

Damit war die Möglichkeit gegeben, das Insert von beiden Seiten her jeweils komplementär zu sequenzieren, und dadurcn die gesamte Nukleotidsequenz des Fragmentes zu erhalten. Die gereinigte Plasmid-DNA von 2,8 SK⁺ und 2,8 SK⁻ wurde nach Pelletierung, Waschen in 75%igem Ethanol und Repelletierung in 10 ul Wasser gelöst, und ein Aliquot von 1 ul davon zur Hengenbestimmung auf einem Gel aufgetragen. Demnach war die Konzentration der DNA 1-2 ug/ul.

### Sequenzierprotokoll:

### 1.) Annealing Reaktion

Annealing-Ansatz: Gesamtvol. 10 ul
2 ul Plasmid-DNA (1-2 ug/ul)
2 ul H₂O
2 ul Sequenzierpuffer (200 mM Tris, pH 7,5, 100 mM MgCl₂, 250 mM NaCl)
1 ul Primer (0,67 pMol/ul)

Die Annealing Mixtur wurde in eine Glaskapillare aufgenommen, die beidendig zugeschmolzen wurde. Diese wurde 5 Minuten im kochenden Wasser behandelt, dann rasch in -70°C kalten Alkohol getaucht, dort 5 Minuten belassen, danach auf 65°C gebracht und dann langsam auf 35°C abgekühlt. Diese Prozedur bewirkt eine Trennung der Doppel stränge und Anlagerung der Primer.

Es wurden 4 Annealing Ansätze hergestellt, je 2 mit Primer und Reverse-Primer.

### 2) Labelling Reaktion

Für die Labelling Reaktion wurde die "Labelling Stock-Lösung" sowohl für die Primer- als auch für die Reverse -Primer-Sequenzierung 1:2 bzw. 1:10 verdünnt. Die 1:2-und 1:10-Ansätze beziehen sich auf die relative Menge der desoxy (d)Nukleotide im Gemisch. Die 1:10-Verdünnung wurde für kurze Strangstücke zur besseren Auflösung der Anfangssequenzen verwendet, die 1:2-Verdünnung für längere Strangstücke.
- Labelling Stock-Lösung:: 7,5 uM dGTP 7,5 uM dCTP 7,5 uM dTTP

### Labelling Ansatz:

Die 4 Annealing Ansätze wurde aus den Kapillaren in Reaktionsgefäße eingebracht.

Zu je 10 ul Annealingmixtur wurden
1 ul 0,1 M Dithiothreit (DTT)
2 ul dNukleotidverdünnung (je 1:2 und 1:10 für Primer und Reverse-Primer)
0,5 ul ³P-alpha-dATP (1 mCi/ml)
2 ul Sequenase (1:8 in TE verdünnt)
zugegeben, gut gemischt, und die 4 Ansätze bei Raumtemperatur 5 Minuten inkubiert.

### 3) Terminationsreaktion

Pro Ansatz wurden 4 Reaktionsröhrchen (A, G, C, T) mit je 2,5 ul "termination mix" (8 uM jedes didesoxy (dd) Nukleotids) auf 37°C gebracht. In diese wurden je 3,5 ul aus der entsprechenden Labelling-Reaktionsansätze pipettiert, was zu einem Einbau der ddNukleotide führt und damit die Strangverlängerung beendet. Die Reaktion wurde nach einer 5 minütigen Inkubation bei 37°C durch Zugabe von je 4 ul einer Stopplösung (98,5% Formamid, 0,05% Bromphenolblau, 0,05% Xylencyanol, 0,5x TE, pH 8) beendet. Die Proben wurden bis zum Auftragen auf das Gel, dem eine 2 minütige Erhitzung der Proben auf 75 bis 85°C zur Strangdenaturierung vorausgeht, bei -20°C aufbewahrt.

### 4) Das Sequenziergel

97,5 ml 6%ige Acrylamid (AA) Stammlösung
2,5 ml 20x TBE Puffer (1 M Tris, pH 7,5, M Borat, 20 mM EDTA)
220 ul Amoniumpersulfat (APDS), 25%ig in H₂O
50 ul Tetramethylethylendiamin (TEMED)
(6%ige AA-Stamm-Lösung:
57 g Acrylamid,
3 g Bisacrylamid,
481 g Harnstoff)
Vor dem Gießen des Gels waren die Glasplatten 3x H₂O, 3x Alkohol gereinigt worden und eine der Platten 2x mit je 5 ml Silanisierlösung (2%iges Dichlormehtylsilan in Chloroform) abgerieben worden. Das Gel polymerisierte über Nacht bei Raumtemperatur.

Das Gel wurde 1 Stunde vor Probenauftragung bei 2000 Volt unter Spannung gesetzt, um eine für die DNA-Naturierung notwendige Temperatur von mindestens 50°C zu schaffen. Die Proben wurden 2 Minuten auf 80°C erhitzt, die Geltaschen gut ausgespült, um Harnstoffpolymerisate zu entfernen. Die Ansätze mit der 1:2 verdünnten dNukleotid "Labelling-Mixture" wurden auf je 2 4-bahnigen Durchläufen (AGCT) aufgetrennt. Zwischen diesen Durchlaufen lag die Zeitspanne zwischen Auftragen und Auslaufen des Blaumarkers und einer weitere Stunde. Nach weiterem 2maligen Auslaufen des Blaumarkers wurde die 1:10 Verdünnung wie oben auf 4 Bahnen aufgetragen und laufen gelassen, bis der Blaumarker den unteren Rand des Gels erreicht hatte. Pro Tasche wurden 2 ul aufgetragen, die Spannung betrug 1500-2000 Volt. Nach Beendigung der Elektrophorese wurde das Gel auf Whatman DEAE-Papier aufgebracht, bei 80°C im Vakuum getrocknet und ohne verstärkerfolie exponiert.

### 5) Analyse der Sequenzdaten

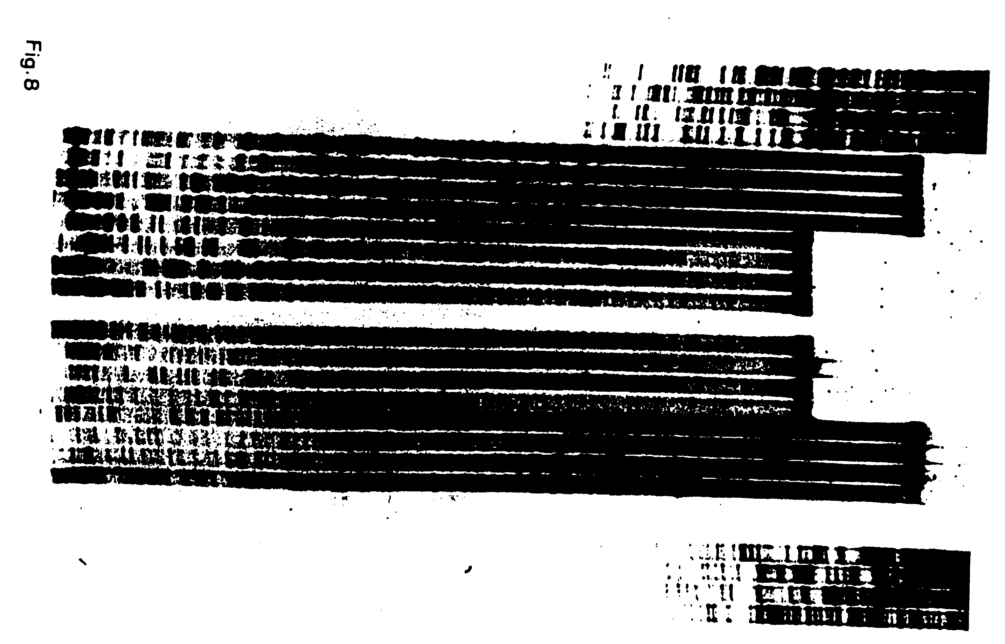

Die sich aus dieser ersten Sequenasereaktion ergebende Sequenz des Inserts was bereits bezüglich der Länge fast vollständig, da die überlappung im mittleren Abschnitt des Inserts vcn beiden Seiten her gelesen werden konnte (Fig. 8). Nach einer Restriktionsanalyse der bereits vorhandenen Sequenzierdaten wurden einige wichtige singuläre 6-Basenschnittstellen ausgewählt, um eine Subklonierung der entsprechenden Fragmente mit anschließender Subsequenzierung vorzunehmen. Dies war notwendig, weil die vorhandene Sequenz noch Unklarheiten aufwies, Widersprüchlichkeiten zwischen komplementären Strangabschnitten zeigte, bzw. Lücken im Anfangsbereich beider Insertenden. Die ausgewählten Schnittstellen waren:
1.) Bcl I: T G A T C A
2.) Bgl II: A G A T C A
3.) BamH I: G G A T C C
Diese drei Stellen weisen untereinander die praktische Beziehung auf, daß sie dieselbe mittlere Basenfolge ..GATC.. aufweisen und nach Restriktion einen 4 Basenüberhang bilden. Daher sind mit diesen Enzymen geschnittene Fragmente untereinander ligierbar.

### 6) Verifizierung der vorhandenen Daten

In Kontrollverdauen wurde die Richtigkeit der Sequenz der Bgl II und BamH I Schnittstelle durch Gelelektrophorese (1% Agarose) bestätigt. Sowohl 2,8 SK⁺- als auch 2,8 SK⁻-Fragmente wurden mit den Enzymkombinationen KpnI/Bgl II, Eco RI/Bgl II bzw. BamH I/Eco RI verdaut. Die erhaltenen Fragmente von ca. 1400 bp, 380 bp bzw. 350 bp waren mit den aus der Genkarte von SK bzw. vom 2,8 kb Fragment errechneten Bandenlängen kompatibel. Abweichungen von ±30 Basenpaaren waren aus Gründen der Sequenzunyollstandigkeit noch möglich.

Zur Bestätigung der erhaltenen Sequenzierdaten bzw. Korrektur der Unklarheiten wurden nun folgende Versuchsanordnungen durchgeführt:

### X. Seouenzierung von Subfragmenten des 2,8 kb Fragmentes nach Maxam & Gilbert

### 1)³P von Subfragmenten nach Bgl II Verdau

Prinzip: Durch Bgl II Verdau entstehen zwei 5'-überhängende Enden. 5'..A..GATCT..3'. Diese werden von der reversen Transkriptase (RT) als Ausgangspunkt für eine ³P-alpha-dATP Markierung von 5'nach 3' verwendet. Anschließendes Nachverdauen mit SacI ergibt 2 radioaktiv markierte Fragmente.

1.) Ein kurzes Bgl II / SacI-Fragment von ca. 1400 bp
2.) Ein langes von Bgl II ausgehend, das Restplasmid umfassende Fragment von ca. 4300 bp.
Bgl II Verdau von 2,8 SK⁺: Ges. vol 200 ul: 10 ug DNA wurden mit high salt Puffer (10 mM Tris, pH 7,5, 10 mM MgCl₂, 100 mM NaCl und 100 ug/ml Rinderserumalbumin) und 60 U Bgl II bei 37°C über Nacht verdaut. Der Verdau wurde phenolisiert (1/4 vol Phenol/Tris saturiert) 2x mit Ether extrahiert und mit Isopropanol gefällt. Nach Pelletierung, Waschen der Pellets in 70%igem Ethanol und Repelletierung wurde die DNA in 20 ul H₂O gelöst. ³P-Markierung der beiden Bgl II Enden: Ges. vol 50 ul
20 ul DNA (1 ug/ul)
5 ul 10x RT Puffer
3 ul ³P-alpha-dATP (1mCi/ml)
5 ul dGTP (10 mM)
1 ul BSA (50 mg/ml)
2 ul RT (20 U/ul)
14 ul H₂O
Das Gemisch wurde 1 Stunde bei 25°C inkubiert.

Anschließend wurde mit SacI nachverdaut. Dazu wurde der RT-Ansatz auf eine Konzentration von 10 mM Tris und 10 mM MgCl₂ eingestellt und damit SacI-kompatibel gemacht. Der Verdau wurde eine Stunde bei bei 37°C inkubiert.

Die Elution der beiden Fragmente wurde nach unter VIII 1 beschriebener Vorschrift aus einem Agarosegel durchgeführt. Nach Phenolisierung und 2-maliger Etherextraktion der wäßrigen Phase erfolgte die Aufteilung der beiden Eluate auf je 5 Reaktionsgefäße mit anschließender Ethanolfällung der markierten Fragmente bei -70°C.

### 2) Sequenzierung nach Maxam & Gilbert

### a) Sequenzierprotokoll

Die DNA-Fragmente (kleines Fragment: ca. 1400 bp, großes Fragment: ca. 4300 bp) wurden mit 70%igem Ethanol gewaschen und pelletiert (je 5 Pellets/Fragment) und nach Maxam & Gilbert sequenziert. Nach Beendigung des Sequenziervorganges wurden die aus den 5 Ansätzen (G, G+A, C+T, C, A+C) erhaltenen Pellets einzeln nach Cerenkov gemessen und danach in unterschiedlichen Voluminamindestens jedoch 12 ul - Formamid-stop-Puffer gelöst, sodaß jedes Gemisch gleich viel Radioaktivität/ul aufwies. Die fertigen Proben wurden bei -20°C aufbewahrt.

### b) Sequenzgele

Durch Auftragen auf Polyacrylamid (PAA)/Harnstoff-Gele mit unterschiedlicher AA-Konzentration (6 %ig bzw. 20 %ig) wurde eine bessere Auflösung in den Anfangssequenzen erreicht.
20 %iges Gel für 60 ml: 20 % AA stock: 197 g AA
1,5 ml 20x TBE Puffer, 3 g BisAA
58,5 ml 20 %ige AA stocksolution 481 g Harnstoff
90 ul 25%iges APDS (Ammoniumperoxodisulfat)
40 ul TEMED (N,N,N',N'-Tetramethylethylendiamin)
2 ul pro Bahn jedes Ansatzes wurden 2 Minuten auf 80°C erhitzt und anschließend auf die Gele aufgetragen. Am 6%igen Gel wurden die Proben - kleines und großes Fragment nebeneinander geführt - in 3 fünfbahnigen (G, G+A, C+T, C, C+A) Durchlaufen bei 2000 Volt aufgetrennt. Zwischen den Durchlaufen lagen beide Male die Zeitspanne bis zum Auslauf des Blaumarkers plus eine Stunde. Am 20 %igen Gel wurde nur jeweils ein fünfbahniger Durchlauf aufgetrennt, bis der Blaumarker in der Gelmitte angelangt war.

### c) Auswertung der aus der Maxam & Gilbert Sequenzierung erhaltenen Daten

Durch diesen zweiten Sequenzierdurchgang konnten die Lücken an den Insertenden ergänzt und ein großer Teil der Unklarheiten beseitigt werden.

### XI. Weitere Subklonierun en als zusätzliche Kontrolle

### 1) Isolierung eines BamHI/HincII-Fragmentes mit Subklonierung und Einzelstransequenzierung durch Sequenase.

Das Ziel war es, die Veränderung der nicht schneidbaren Eco RI-Schnittstelle genau zu beschreiben. Deshalb wurde eine singuläre HincII-Schnittstelle ausgewählt, die 126 Basen außerhalb dieser Eco RI-Schnittstelle entfernt auf dem der SacI-Schnittstelle zugewandten lambda-gt11-Arm liegt.
a) BamHI/HincII Verdau
   i) von 2,8 SK⁺: 3 ug DNA wurden mit je 15 U BamHI und HincII in high salt Puffer (HS) verdaut,
   ii) von SK⁺ und SK⁻: je 2 ug SK⁺ bzw. SK⁻ wurden von je 10 U BamHI und HincII in HS Puffer verdaut. Die Inkubation erfolgte bei 37°C über Nacht.
   Nach Gelelektrophorese wurde das BamHI/HincII Fragment eluiert (s. unter VIII 1 b). Eine Hälfte des Eluates wurde mit dem SK⁺-Verdau, die andere Hälfte mit dem SK⁻-verdau vereinigt, und beide DNA-Gemische mit Ethanol gefällt.
b) Nach Pelletierung der beiden DNA Fällungen erfolgten Ligation und Transformation in XLI blue (wie unter VIII 2 b und c beschrieben).

### 2.) Isolierung eines Eco RI/Bgl II-Fragmentes mit Subklonierung und Finzelstrangsequenzierung durch Sequenase

a.) Eco RI/Bgl II-Verdau
   i) von 2,8 SK⁺: 12 ug 2,8 SK⁺ DNA wurden mit je 60 U Eco und Bgl II in HS Puffer verdaut
   ii) Von SK⁺ und SK⁻: je 2 ug SK⁺ bzw. SK⁻ wurden von 10 U BamHI und 10 U Bgl II in HS Puffer verdaut. Die Inkubationen wurden über 3 Stunden bei 37°C durchgeführt.
   Die zu i) korrespondierenden Verdaue von SK⁺ und SK⁻ wurden mit Eco RI und BamHI durchgeführt (das Bluescriptplasmid besitzt keine Egl II Schnittstelle am Polylinker). Dabei wurde die Ligierbarkeit der beiden palindromischen Schnittstellen verwertet.
   Nach Gelelektrophorese wurde das Eco RI/Bgl II Fragment eluiert (wie unter VIII 1 b) und mit den Eco RI/BamHI verdauten SK⁺- und SK⁻-Plasmiden (wie unter XI 1 a beschrieben) mit Ethanol gefällt.
b.) Ligation und Tansformation erfolgten wie unter VIII 2 b und c beschrieben.

### Verifizierung der in XI 1 und XI 2 durchgeführten Transformationen.

Durch Plasmidpreparation nach Methode der alkalischen Lyse wurde aus den Transformanten beider Ligationen die Subfragment-tragenden SK⁺- und SK⁻-Bluescript-Plasmide isoliert.

### Kontrollverdaue der Rekombinanten:

1.) BamHI/HincII zur Bestätigung der BamHI/HincII-Insertion
2.) Eco RI/XbaI zur Bestätigung der Eco RI/Bgl II-Insertion (die Bgl II-Stelle war durch Ligierung mit der BamHI-Schnittstelle nicht mehr schneidbar)
   Beide Verdaue wurden auf Agarose-Gelesen aufgetrennt. Dadurch konnten Ligation und Transformation beider Subfragmente in SK⁺ bzw. SK⁻ dokumentiert werden. Gleichzeitig konnten die Ausgangsklone für die Einzelstrangsequenzierung nach qualitativen und quantitativen Kriterien ausgewählt werden.

### 3) Einzelstrangsequenzierung von Bluescript-DNA-Subfragmenten

### a) Herstellung von Einzelstrang-DNA durch Helfer-Phagen

Je ein SK⁺ und SK⁻ Subklon mit dem BamHI/HincII- bzw. dem Eco RI/BglII-Fragment wurde in 2,5 ml Vorkultur (LB/amp/tet) über Nacht bei 37°C vermehrt. Daraus wurden mit je 50 ul wieder 2,5 ml LB beimpft. Nach 30 Minuten bei 37°C wurden je 10 ul des Bluescript- Helferphagen R408 mit einem Titer von 5,5 x 10(10) plaque forming units/ml zugegeben und die 4 Kulturen 8 Stunden bei 37°C geschüttelt. Anschließend wurden je 1,2 ml in Reaktionsröhrchen transferiert und 15 Minuten bei 15 000 g zentrifugiert. Die Einzelstrangvarianten der Insert-tragenden Plasmide
Eco RI/BamHI SK⁺ ligiert mit Eco RI/Bgl II-Fragment
Eco RI/BamHI SK⁻ ligiert mit Eco RI/Bgl II-Fragment
HincII/BamHI SK⁺ ligiert mit HincII/BamHI-Fragment
HincII/BamHI SK⁻ liegiert mit HincII/BamHI-Fragment
waren nun im überstand und wurden mit 300 ul Polyethylenglycol (20%igem PEG 6000 in 3,5 M Ammonacetat) gefällt. Nach 15 Minuten bei Raumtemperatur erfolgte Pelletierung und Lösen der Einzelstrang DNA in 300 ul TE Puffer. Nach Phenol- und 2maliger Etherextraktion der wäßrigen Phase erfolgte die Ethanolfällung unter Zusatz von 50 vol% 7,5 M Ammonacetat bei -70°C.

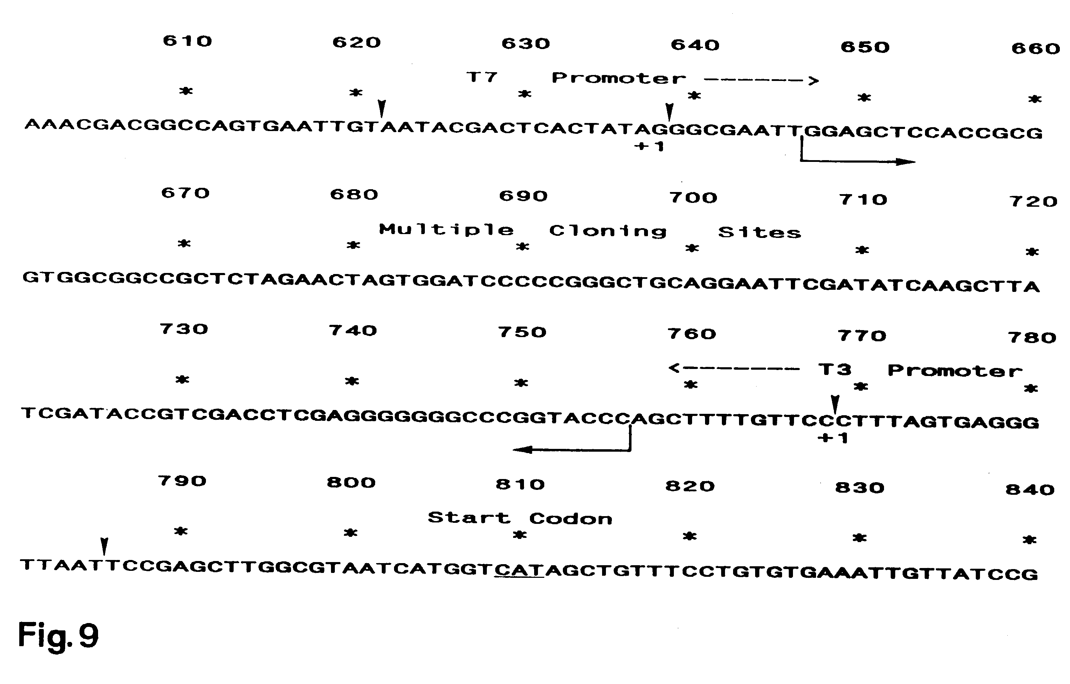

Als Primer für das Einzelstrangsequenzieren mit Sequenase wurden 2 käufliche Oligonukleotide verwendet, die Abschnitten des Bluescript-LacZ Gens zu beiden Seiten des Polylinkers entsprechen.
1.) T3-Primer: ein 17 Basen langes Oligonukleotid mit der Sequenz 5'.....A T T A A C C C T C A C T A A A G .... 3' auf der SacI-Seite des SK LacZ-Genes dem Polylinker vorgeschaltet, für die Primingreaktion mit den SK⁻-Rekombinanten (Fig. 9).
2.) T7-Primer: ein 17 Basen langes Oligonukleotid mit der Sequenz 5'.... A A T A C G A C T C A C T A T A G .....3' auf der KpnI-Seite dem Polylinker vorgelagert, für die Primingreaktion mit den SK⁺-Rekombinanten (Fig. 9).

### c) Sequenzierprotokoll

Die Einzelstrang DNA wurden nach der PEG-Behandlung mit 70%igem Ethanol gewaschen, pelletiert und in 10 ul H₂O gelöst. Nach Gelkontrolle von Aliquoten (1 ul) wurden je 7,5 ul DNA mit etwa 400-600 ng/Ansatz in die Annealingreaktion eingesetzt. Die dNukleotidverdünnung für die Labellingreaktion wurde mit 1,5 (dNukleotid-Stammlösung in H₂O) angesetzt, die Markierung erfolgte mit ³P-alpha-dATP. Alle weiteren Schritte waren identisch mit den bereits in der ersten Sequenasesequenzierung beschriebenen. Die Terminationsgemische wurden in je 2 vierbahnigen (ACGT) Durchläufen, zwischen denen jeweils die Zeitspanne für das zweimalige Auslaufen des Blaumarkers lag, aufgetrennt. Es wurde ein 6 %iges Gel verwendet.

### d) Auswertung der nach diesem dritten Sequenzierdurchgang erhaltenen Daten.

Die durch die Einzelstrang-Sequenzierung durch Sequenase nunmehr vollständige dreifach kontrollierte Nukleotidsequenz des Hauptallergens der Birke *Bet v* I ist Fig. 10 zu entnehmen.

Die Länge des cDNA Inserts beträgt 727 Basen (Fig. 10). Die Basen 1-11, d.h. die Basen bis zur Schnittstelle der (T-deletierten) Eco RI-Stelle an den Positionen 11-15, und die Basen 739-744, d.h. die Basen von der Schnittstelle der (intakten) Eco RI-Stelle bis zum Sequenzende, stammen vom Phagen lambda gtll.

### 1) Der kodierende Abschnitt

Die für *Bet v* I kodierende Sequenz ist in Fig. 10 durch Beginn und Ende der zur Nukleotidsequenz parallel laufenden Aminosäuresequenz gekennzeichnet. Das kodierte Protein weist ein errechnetes MG von 17.570 auf. Die Sequenz beginnt mit dem für die Aminosäure (AS) Methionin kodierenden Basentriplett ATG (Position 65-67) und endet mit dem Basentriplett TAA (Position 545-547). Diese beiden Tripletts sind durch den genetischen Code als Anfangs- und Endcodons definiert. Von ATG bis TAA ist der Leserahmen offen.

Die aus dieser Nukleotidsequenz erhaltene Proteinsequenz stimmt überein mit der AS-Sequenz des Proteins *Bet v* I, das bis zur AS 35 vom N-Terminus sequenziert wurde.

Die Basentripletts von Position 311 bis Position 319 kodieren für die AS: Asn-Tyr-Ser. Es handelt sich bei diesem AS-Triplett um eine potentielle Glykosylierungsstelle.

Die DNA-Sequenz von *Bet v* I zeigt hohe Homologie mit einem Krankheitsresistenz hervorrufenden Gen der Erbse, wie dies bereits in der Einleitung erörtert wurde.

### 2) Nicht-kodierende Abschnitte

### a) Der nicht-kodierende Abschnitt von Base 12 bis Base 64

Die Veränderung der Eco RI-Stelle an Position 11 wurde durch eine Deletion eines Thymidin hervorgerufen.

Bei den unmittelbar dem Startcodon vorangehenden Basen: GCC ATC ATG handelt es sich um eine in der Literatur beschriebene Signalsequenz, wobei das in minus-3-Position stehende A konstant ist, die anderen Basen aber differieren können (Lutcke et al., EMBO J. 6, 43-48, 1987).

### b) Der nicht-kodierende Abschnitt von Base 548 bis 744

Das Ende der cDNA ist durch eine poly-Adenylsequenz von 29 A charakterisiert.

Die Basensequenz von Position 693-698 AAT AAA. ist als eine für die Polyadenylierung wesentliche Konsensus-Signalsequenz in der Literatur beschrieben (M. Birnstiel et al., Cell 41, 349, 19..). Aus obigen Daten geht hervor:

Es wurde die gesamte mRNA über cDNA kloniert. Der Leserahmen ist offen. Es handelt sich bei oben angeführter Sequenz um das vollständige Gen des Proteins *Bet v* I*.*

### Zusammenfassende Legenden zu den beiliegenden Figuren

Fig. 1. RNA-Charakterisierung auf 6% Polyacrylamid - 50% Harnstoffgel. M: Marker. 1-5: unabhängige RNA Isolationen aus männlichen Infloreszenzen.
Fig. 2. Detektion positiver Insert-tragender Klone mittels IgE-Antikörpern aus Allergikerseren.
   1) 1. Detektionsschritt: 2 positive Klone (Pfeil) weitergezogen
   2) Reklonierung der positiven Klone
Fig. 3. Restriktionskarte des Phagen lambda gt11. Pfeil (mit Stern) markiert die Insertionsstelle für die cDNA.
Fig. 4 A.
   1) lambda gt11 DNA mit Insert (KpnI/SacI-verdaut): 2,8 kbp KpnI/SacI-Fragment (Karo).
   2) lambda gt11 DNA ohne Insert (KpnI/SacI-verdaut): 2,08 kbp KpnI/SacI-Fragment (Karo).
   3) lambda gt11 DNA mit Insert (SacI/Eco RI-verdaut): 1,06 kbp SacI/Eco RI-Fragment (Doppelkaro).
   4) lambda gt11 DNA ohne Insert (SacI/Eco RI-verdaut): 1,8 kbp SacI/Eco RI-Fragment (Doppelkaro).
   5) lambdaWildtyp-DNAPstI verdaut.
Fig. 4 B. Ausschnitt aus dem lambda gt11 Genom mit dem cDNA-Insert, den beiden Eco RI-Schnittstellen (die der SacI-Stelle näher liegende ist deletiert) und den flankierenden KpnI- und SacI-Schnittstellen. Der Anteil des lac Z-Gens ist durch Punkte unterlegt.
Fig. 5. Western-/Immunoblot
   A) Immunoblot mit Allergikerserum; Detektion mit ¹⁵J-anti IgE.
      1) Proteinextrakt aus *E. coli* Y 1089
      2) Proteinextrakt aus *E. coli* Y 1089 infiziert mit Lambda gt11 ohne Insert
      3) Proteinextrakt aus *E. coli Y* 1089 infiziert mit lambda gt11 Insert-tragend (Klon HB6)
      4) Proteinextrakt aus *E. coli Y* 1089 infiziert mit lambda gt11 Insert-tragend (Klon HB8)
      5) Birkenpollenextrakt BP VIII D
   B) Immunoblot mit monoklonalem Antikörper Bip 1; Detektion mit anti-Maus IgG (Kaninchen) und ¹⁵J-anti-Kaninchen Ig.
      1) Proteinextrakt aus *E. coli Y* 1089
      2) Proteinextrakt aus *E. coli Y* 1089 infiziert mit lambda gt11 ohne Insert
      3) Proteinextrakt aus *E. coli Y* 1089 infiziert mit lambda gt11 Insert-tragend (Klon HB6)
      4) Proteinextrakt aus *E. coli Y* 1089 infiziert mit lambda gt11 Insert-tragend (Klon HB8)
      5) Birkenpollenextrakt BP VIII D
Fig. 6. Restriktionskarte des Plasmides pBluescript SK(+/-).
Fig. 7. Ausschnitt aus dem 2,8 kb-Fragment zur Darstellung des cDNA-Inserts mit lambda gt11-Sequenzier-Primern; die Pfeile weisen in Syntheserichtung.
Fig. 8. Autoradiographie des Sequenziergels nach der ersten Sequenzierung des cDNA-Inserts mit Hilfe der Lambda-gt11-Primer und Sequenase; radioaktives Isotop: ³P.
Fig. 9. Ausschnitt aus dem lacZ-Gen des Bluescriptplasmids. Dargestellt ist der Bereich vom T7-Promotor bis zum T3-Promotor. Dazwischen liegt der Polylinker. Anfangs und Ende der beiden Primer sind durch Pfeile angedeutet.
Fig. 10. Darstellung der endgültigen Sequenz des cDNA-Inserts.

## Patentansprüche

1. Verfahren zur Auffindung von Polynukleotiden, die in ihrem offenen Leserahmen für Proteine kodieren, deren biologische Aktivität als Allergene den in der Natur vorkommenden Pflanzenallergenen entsprechen, dadurch gekennzeichnet, daß aus Pflanzengewebe eine cDNA-Klonbank erzeugt wird, daß durch Expression dieser Klonbank Proteine erzeugt werden, welche mit Allergikerseren zur Ermittlung der mit IgE-Antikörpern reagierenden Proteine in Kontakt gebracht werden, und daß allergene-Fusionsproteine nachgewiesen werden mittels Bindung von anti-IgE an allergene-Fusionsproteine/IgE complexen.

2. Verfahren zur Herstellung eines im Verfahren nach Anspruch 1 einsetzbaren Reaktanten, dadurch gekennzeichnet, daß das für das identifizierte Protein kodierende Polynukleotid in lambda-gt11-Phagen inseriert wird.

3. Verfahren zur Herstellung eines im Verfahren nach Anspruch 1 einsetzbaren Reaktanten, dadurch gekennzeichnet, daß das für das identifizierte Protein kodierende Polynukleotid in das Plasmid "Bluescript SK (+/-)" inseriert wird.

4. Verfahren nach Anspruch 2 oder 3, dadurch gekennzeichnet, daß der erzeugte Vektor in einem einzelligen Mikroorganismus, insbesondere E.coli, zur Expression gebracht wird.

5. cDNA die nach dem Verfahren nach Anspruch 1 gescreent wurde, dadurch gekennzeichnet, daß sie für die durch die Sequenz festlegten allergen wirksamen Epitope des Proteins kodiert, wobei sie dem affenen Leserahmen der in Fig 10 wiedergegebenen Sequenz entspricht.

6. cDNA nach Anspruch 5, dadurch gekennzeichnet, daß sie die in Fig. 10 wiedergegebene Sequenz oder Teilsequenzen daraus aufweist.

7. cDNA nach Anspruch 5 oder 6, dadurch gekennzeichnet, daß sie eine der mit der Sequenz gemäß Fig. 10 unter stringenten Bedingungen hybridisierende Sequenz aufweist und für das nach dem Verfahren gemäß Anspruch 1 identifizierte Protein kodiert.

8. cDNA nach Anspruch 5 oder 6, dadurch gekennzeichnet, daß sie eine durch Degeneration der Sequenz gemäß Fig. 10 abgeleitete Sequenz aufweist und für das nach dem Verfahren gemäß Anspruch 1 identifizierte Protein kodiert.

## Claims

1. A method for detecting polynucleotides which encode in their open reading frame proteins whose biological activity as allergens correspond to the plant allergens found in nature, characterized in that a cDNA clone bank is produced from plant tissue, that proteins are produced by expression of this clone bank which are brought into contact with sera of allergic persons for determining the proteins reacting with IgE antibodies and that allergenic fusion proteins are detected by means of bonding of anti-IgE to allergenic fusion proteins/IgE complexes.

2. A method for producing a reactant which is usable in the method as claimed in claim 1, characterized in that the polynucleotide encoding the identified protein is inserted into lambda gt11 phages.

3. A method for producing a reactant which is usable in the method as claimed in claim 1, characterized in that the polynucleotide encoding the identified protein is inserted into the plasmid "Bluescript SK (+/-)".

4. A method as claimed in claim 2 or 3, characterized in that the produced vector is expressed in a unicellular microorganism, in particular E. coli.

5. A cDNA which was screened according to the method as claimed in claim 1, characterized in that it encodes the allergenically effective epitopes of the protein which are determined by the sequence, with the cDNA corresponding to the open reading frame of the sequence as shown in Fig. 10.

6. A cDNA as claimed in claim 5, characterized in that it comprises the sequence or partial sequence as represented in Fig. 10.

7. A cDNA as claimed in claim 5 or 6, characterized in that it comprises a sequence hybridizing under stringent conditions with the sequence according to Fig. 10 and encodes the protein identified in accordance with the method as claimed in claim 1.

8. A cDNA as claimed in claim 5 or 6, characterized in that it comprises a sequence derived by degeneration of the sequence according to Fig. 10 and encodes the protein identified in accordance with the method as claimed in claim 1.

## Revendications

1. Procédé de détection de polynucléotides, lesquels codent dans leur cadre de lecture ouvert pour des protéines dont l'activité biologique comme allergènes correspond à celles des allergènes végétaux naturels, caractérisé en ce qu'on produit une banque de clones d'ADNc à partir de tissus végétaux, en ce qu'on produit des protéines par expression de cette banque de clones, lesquelles sont mises en contact avec des sérums d'allergiques afin de déterminer les protéines réagissant avec des anticorps IgE et en ce qu'on met en évidence des protéines de fusion allergènes au moyen d'une liaison d'anti-IgE avec des complexes protéines de fusion allergènes/IgE.

2. Procédé pour la fabrication d'un réactif pouvant être utilisé dans le procédé selon la revendication 1 caractérisé en ce que le polynucléotide codant pour la protéine identifiée est inséré dans des phages lambda-gt11.

3. Procédé pour la fabrication d'un réactif pouvant être utilisé dans le procédé selon la revendication 1 caractérisé en ce que le polynucléotide codant pour la protéine identifiée est inséré dans le plasmide "Bluescript SK (+/-)".

4. Procédé selon la revendication 2 ou 3 caractérisé en ce qu'on amène le vecteur produit à s'exprimer dans un microorganisme unicellulaire, notamment Escherichia coli.

5. ADNc examiné selon le procédé de la revendication 1 caractérisé en ce qu'il code pour les épitopes actifs comme allergènes de la protéine déterminés au moyen de la séquence, l'ADNc correspondant au cadre de lecture ouvert de la séquence représentée à la figure 10.

6. ADNc selon la revendication 5 caractérisé en ce qu'il présente la séquence représentée à la figure 10 ou des parts de cette séquence.

7. ADNc selon la revendication 5 ou 6 caractérisé en ce qu'il présente une séquence hybridant avec la séquence selon la figure 10 dans des conditions rigoureuses et qu'il code pour la protéine identifiée selon le procédé conforme à la revendication 1.

8. ADNc selon la revendication 5 ou 6 caractérisé en ce qu'il présente une séquence dérivée par dégénération de la séquence selon la figure 10 et qu'il code pour la protéine identifiée selon le procédé conforme à la revendication 1.
